# EUROPÄISCHE PATENTSCHRIFT

(11) **EP 1 109 769 B1**
(45) Veröffentlichungstag und Bekanntmachung des Hinweises auf die Patenterteilung: **03.07.2002**
(21) Anmeldenummer: 99946008.2
(22) Anmeldetag: 23.08.1999
(51) Int. Cl.: C07C 39/12

(54) **VERFAHREN ZUR HERSTELLUNG VON BIS(4-HYDROXYARYL)ALKANEN**
METHOD FOR PRODUCING BIS(4-HYDROXYARYL)ALKANES
PROCEDE PERMETTANT DE FABRIQUER DES BIS(4-HYDROXYARYL)ALCANES

(30) Priorität: 03.09.1998 DE 19840110
(43) Veröffentlichungstag der Anmeldung: 27.06.2001
(73) Patentinhaber: Bayer Aktiengesellschaft, 51368 Leverkusen (DE)
(72) Erfinder: KÜHLING, Steffen, B-9100 Sint Niklaas (BE); LANZE, Rolf, D-47804 Krefeld (DE); NEUMANN, Rainer, D-47803 Krefeld (DE); HEYDENREICH, Frieder, D-40593 Düsseldorf (DE); VAN OSSELAER, Tony, D-47800 Krefeld (DE); FENNHOFF, Gerhard, D-47877 Willich (DE)
(86) Internationale Anmeldenummer: EP9906146
(87) Internationale Veröffentlichungsnummer: WO0014044

(56) Entgegenhaltungen:
- EP-A- 0 499 922

## Beschreibung

Die vorliegende Erfindung betrifft ein Verfahren zur Herstellung von Bis(4-hydroxyaryl)alkanen hoher Reinheit aus Addukten von Bis(4-hydroxyaryl)alkanen und aromatischen Hydroxyverbindungen, die durch sauer katalysierte Umsetzung der aromatischen Hydroxyverbindungen mit Ketonen erhalten werden.

Die Synthese von Bis(4-hydroxyaryl)alkanen durch sauer katalysierte Umsetzung aromatischer Hydroxyverbindungen mit Ketonen ist bekannt, beispielsweise aus US-A 2 775 620 oder EP-A 342 758. In der Regel wird als Zwischenprodukt ein Addukt des Bis(4-hydroxyaryl)alkans und der als Edukt eingesetzten aromatischen Hydroxyverbindung erhalten, das anschließend destillativ von der aromatischen Hydroxyverbindung befreit wird. Das großtechnisch wichtigste Beispiel ist die Herstellung von Bisphenol A, bei der intermediär ein Addukt von Bisphenol A (BPA) und Phenol erhalten wird. Auch nach Reinigung, beispielsweise durch Umkristallisation, enthält dieses Addukt aufgrund der sauer katalysierten Herstellung noch Spuren von Säure (ca. 5 - 10·10⁻⁶ mol Säure/mol BPA). Diese Säurespuren führen bei der Abtrennung des Phenols vom Bisphenol A, die mit einer thermischen Belastung verbunden ist, zu teilweiser Zersetzung des Bisphenols und der Bildung von Nebenprodukten. Folge dieser Zersetzungsreaktionen sind die Verschlechterung der Reinheit und Farbqualität des Bisphenols. Dies wirkt sich auch auf die Qualität der aus den Bisphenolen hergestellten Produkte wie Epoxidharze, Polyester, Polyestercarbonate und Polycarbonate negativ aus, und zwar dergestalt, daß Farbprobleme, schlechte Lichtdurchlässigkeit transparenter Produkte oder Stippen in den Oberflächen von Formteilen aus diesen Endprodukten die Folge sind. Analoge Verhältnisse treten auch bei der Herstellung anderer Bis(4-hydroxyaryl)alkane auf. Deshalb ist es von besonderem Interesse, die thermische Stabilität der Bis(4-hydroxyaryl)alkane und ihrer Addukte mit aromatischen Hydroxyverbindungen zu verbessern.

Verschiedene Verfahren zur Stabilisierung von Bis(4-hydroxyaryl)alkanen sind bereits bekannt geworden. Nach EP-A 374 692 werden Hydroxycarbonsäuren bzw. deren Ammonium- oder Alkalisalze als Additive eingesetzt. Gemäß EP-A 523 931 werden dem jeweiligen Addukt aus Bis(4-hydroxyaryl)alkan und aromatischer Hydroxyverbindung Alkalisalze aliphatischer Carbonsäuren zugesetzt. US-A 5 399 789 schlägt vor, den Bis(4-hydroxyaryl)alkanen vor der thermischen Aufarbeitung Amine in Mengen von 1 bis 1000 ppm zuzusetzen. Allen genannten Verfahren ist gemeinsam, daß eine Überdosierung der basischen Substanzen zu Produktzersetzung im thermischen Auf- und Weiterverarbeitungsprozeß führt.

EP-A 469 689 beschreibt ein Verfahren, bei dem die adsorptive Wirkung von Aktivkohle genutzt wird, um das Bis(4-hydroxyaryl)alkan vor Spaltungsprozessen in den nachfolgenden Aufarbeitungsstufen zu bewahren. Hohe Durchsätze von Reaktionsgemisch, insbesondere bei kontinuierlicher Betriebsweise, führen jedoch selbst bei Verwendung von Aktivkohlegranulaten zu Feinstabrieb der Aktivkohle, der immer wieder, auch bei sorgfältigster Filtration, bis ins Endprodukt gelangen kann und dessen farbliche Qualität und Reinheit verschlechtert.

In EP-A 559 372 wird die Kombination eines sauren und eines basischen Ionentauscherharzes in Form eines Mischbetts beschrieben, durch welches Mutterlauge geleitet und so gereinigt wird, bevor sie dem Eduktgemisch vor der eigentlichen Reaktion zugesetzt wird. Dieses Verfahren hat jedoch den Nachteil, daß zersetzend wirkende Substanzen, die erst im Verlauf der Reaktion gebildet werden, nicht entfernt werden und so weiterhin die Produktqualität verschlechtern können. Zudem gelangen stets Oligomere dieser Harze in gelöster Form in den Produktstrom und beeinträchtigen ebenfalls die Produktqualität.

EP-A 0 499 922 offenbart ein Verfahren zur Reinigung von Bisphenolen, die als Bisphenol-Phenol-Adukt anfallen, durch Destillation.

Aufgabe der Erfindung war es, ein Verfahren bereitzustellen, nach dem Bis(4-hydroxyaryl)alkane mit einem geringeren Anteil an Nebenprodukten erhalten werden können. Es wurde nun ein Verfahren gefunden, bei dem ein neutralisierendes Agens in sehr feinverteilter Form auf das Bis(4-hydroxyaryl)alkan-Addukt aufgebracht wird. In einer bevorzugten Ausführungsform des erfindungsgemäßen Verfahrens wird die Dosierung des neutralisierenden Agens in Abhängigkeit vom Isomerengehalt des Produkts geregelt.

Gegenstand der Erfindung ist ein Verfahren zur Herstellung von Bis(4-hydroxyaryl)alkanen, bei dem einem durch sauer katalysierte Umsetzung aromatischer Hydroxyverbindungen mit Ketonen erhaltenen Addukt von Bis(4-hydroxyaryl)alkan und der als Edukt eingesetzten aromatischen Hydroxyverbindung wäßrige Alkalihydroxid-Lösung, z.B. wäßrige NaOH, als Aerosol über die Gasphase zugeführt wird und das Addukt anschließend destillativ von der aromatischen Hydroxyverbindung befreit wird.

In einer bevorzugten Ausführungsform handelt es sich bei dem Addukt um das Addukt von Bisphenol A und Phenol.

Die Neutralisation der im Addukt herstellungsbedingt noch enthaltenen Säurespuren bereitet im großtechnischen Verfahren extreme Schwierigkeiten, da nur sehr kleine Mengen an Base erforderlich sind, die gleichmäßig über das kristalline Addukt verteilt werden müssen. Erfindungsgemäß wird diese Schwierigkeit dadurch überwunden, daß wäßrige Alkalihydroxid-Lösung als Aerosol auf das Addukt aufgebracht wird. Über die Konzentration der vorgelegten Alkalihydroxid-Lösung wird die Menge an Alkalihydroxid, die auf das Addukt aufgebracht wird, geregelt. Die Konzentration beträgt hierbei bevorzugt 0,005 bis 0,015 Gew.-% Alkalihydroxid.

Eine weitere Schwierigkeit ist die Steuerung der Neutralisation, da eine pH-Messung am Addukt zur Bestimmung des Neutralpunkts bislang technisch nicht möglich ist. In einer bevorzugten Ausführungsform der Erfindung, einem kontinuierlichen Herstellungsverfahren für Bis(4-hydroxyaryl)alkane, wird dieses Problem dadurch gelöst, daß dem kristallin anfallenden und von der Mutterlauge befreiten Addukt ein konstanter Massenstrom von Alkalihydroxid zugeführt wird, das Addukt anschliessend von der enthaltenenen aromatischen Hydroxyverbindung befreit, der Nebenproduktgehalt im erhaltenenen Bis(4-hydroxyaryl)alkan analytisch bestimmt und, falls der Gehalt bestimmter Nebenprodukte einen vorgegebenen Schwellenwert überschreitet, der Massenstrom von Alkalihydroxid entsprechend nachgeregelt wird. Die Bestimmung der Nebenprodukte im Bis(4-hydroxyaryl)alkan erfolgt bevorzugt gaschromatografisch oder durch Hochdruckflüssigkeitschromatographie. Die Bestimmung der Nebenprodukte kann auch on-line erfolgen. Dadurch kann das Regelungsintervall verkürzt werden und Schwankungen der Produktzusammensetzung lassen sich minimieren. Das Alkalihydroxid wird bevorzugt als Aerosol einer wäßrigen Alkalihydroxid-Lösung zugeführt und der Massenstrom von Alkalihydroxid über die Konzentration der Alkalihydroxid-Lösung geregelt. In einer besonders bevorzugten Ausführungsform wird verdünnte Alkalihydroxid-Lösung als Betriebsflüssigkeit einer Wasserringpumpe eingesetzt und dadurch auf der Druckseite ein Aerosol erzeugt. Durch kontinuierliche mengengeregelte Zufuhr von Wasser und Alkalihydroxid-Lösung und kontinuierliche Ausschleusung eines Teils der Betriebsflüssigkeit kann die Alkalihydroxid-Konzentration in der Betriebsflüssigkeit und damit im Aerosol geregelt werden. Die Pumpe wirkt außerdem als Mischaggregat und sorgt für eine homogene Durchmischung der Betriebsflüssigkeit.

Das erfindungsgemäße Verfahren eignet sich zur Herstellung von Bis(4-hydroxyaryl)-alkanen auf der Basis von Ketonen mit wenigstens einer aliphatischen Gruppe an der Carbonylfunktion; genannt seien beispielsweise Aceton, Methylethylketon, Methylpropylketon, Methylisopropylketon, Diethylketon, Acetophenon, Cyclohexanon, Cyclopentanon, Methyl-, Dimethyl- und Trimethylcyclohexanone, die auch geminale Methylgruppen aufweisen können wie 3,3-Dimethyl-5-methylcyclohexanon (Hydroisophoron). Bevorzugt sind Aceton, Acetophenon, Cyclohexanon und dessen Methylgruppen tragende Homologe; besonders bevorzugt ist Aceton.

Zur Herstellung der im erfindungsgemäßen Verfahren eingesetzten Addukte aus Bis-(4-hydroxyaryl)alkan und aromatischer Hydroxyverbindung geeignete aromatische Hydroxyverbindungen sind in p-Position nicht substituiert und enthalten keine Substituenten zweiter Ordnung wie Cyano-, Carboxy- oder Nitrogruppen; genannt seien beispielsweise Phenol, o- und m-Kresol, 2,6-Dimethylphenol, o-tert.-Butylphenol, 2-Methyl-6-tert.-Butylphenol, o-Cyclohexylphenol, o-Phenylphenol, o-Isopropylphenol, 2-Methyl-6-cyclopentyl-phenol, o- und m-Chlorphenol, 2,3,6-Trimethylphenol. Bevorzugt sind Phenol, o- und m-Kresol, 2,6-Dimethylphenol, o-tert.-Butylphenol und o-Phenylphenol; besonders bevorzugt ist Phenol.

### Beispiele

### Beispiel 1

Aus Phenol und Aceton wird in einem kontinuierlichen Prozeß durch sauer katalysierte Umsetzung ein kristallines Bisphenol A/Phenol-Addukt erhalten, das durch Filtration von der Mutterlauge abgetrennt wird. Dem kristallisierten BPA/ Phenol-Addukt wird kontinuierlich über die Gasphase die zur Neutralisation erforderliche Menge NaOH als Aerosol zugeführt. Über die Konzentration der vorgelegten wäßrigen NaOH-Lösung wird die Menge an NaOH geregelt; die auf das BPA/Phenol-Addukt aufgebracht wird; diese Konzentration beträgt 0,005 bis 0,015 Gew.-% NaOH. Die Einstellung der Konzentration erfolgt mengengeregelt über die Verdünnung einer 6,5 Gew.-%igen NaOH mit VE-Wasser. Das nach dem thermischen Abtrennvorgang des Phenols resultierende BPA wird im Abstand von 4 Stunden gaschromatografisch analysiert. Die Regelung der NaOH-Konzentration erfolgt in Abhängigkeit vom Gehalt an Isomeren im BPA. Beträgt der Gehalt an Isopropenylphenol und dessen Dimeren in der Summe weniger als 100 ppm, so wird die Konzentration der NaOH unverändert gelassen, bei höheren Gehalten wird die NaOH-Konzentration erhöht. Bei Dimerkonzentrationen < 10 ppm und erhöhten Gehalten an Trisphenol (> 300 ppm), die auf eine basische Zersetzung hinweisen, wird die NaOH-Konzentration erniedrigt. Mit dieser Verfahrensweise können die Dimerenund Isopropenylphenolgehalte des hergestellten Bisphenols auf Werte < 100 ppm begrenzt werden. Durch die kontinuierliche NaOH-Dosierung, die nach der GC-Analytik (Dimeren-, Isopropenylphenol- und Trisphenolgehalte) geregelt wird, kann eine wesentlich konstantere und höhere BPA-Qualität erzielt werden.

### Vergleichsbeispiel 2

Dem kristallisierten BPA/Phenol-Addukt wird keine NaOH über die Gasphase zugeführt. Der Gehalt an Isopropenylphenol und dessen Dimeren im BPA steigt in Summe bis auf 900 ppm an. Eine hohe BPA-Qualität mit niedrigen Isomerengehalten (Dimeren-, Isopropenylphenol- und Trisphenol) wird nicht erreicht.

### Vergleichsbeispiel 3

Dem kristallisierten BPA/Phenol-Addukt wird über die Gasphase diskontinuierlich alle 24 h eine Menge NaOH zugeführt, die der in Beispiel 1 im Zeitraum von 24 h zugeführten Menge NaOH entspricht. Als Resultat wird ein stark variierender Gehalt an Isopropenylphenol, dessen Dimeren und an Trisphenol im BPA beobachtet. Der Gehalt an Dimeren und Isopropenylphenol schwankt zwischen < 100 und 700 ppm, der Gehalt an Trisphenolen zwischen 250 und 500 ppm. Eine konstante BPA-Qualität mit niedrigen Isomerengehalten wird nicht erhalten.

## Patentansprüche

1. Verfahren zur Herstellung von Bis(4-hydroxyaryl)alkanen aus Addukten von Bis(4-hydroxyaryl)alkanen und aromatischen Hydroxyverbindungen, die durch sauer katalysiere Umsetzung der aromatischen Hydroxyverbindungen mit Ketonen erhalten werden, bei dem dem kristallinen Addukt wäßrige Alkalihydroxid-Lösung als Aerosol über die Gasphase zugeführt wird und das Addukt anschließend destillativ von der aromatischen Hydroxyverbindung befreit wird.

2. Verfahren gemäß Anspruch 1, bei dem das Addukt von Bis(4-hydroxyaryl)alkan und aromatischen Hydroxyverbindung ein Addukt von Bisphenol A und Phenol ist.

3. Verfahren gemäß Anspruch 2, bei dem als wäßrige Alkalihydroxidlösung 0,005 bis 0,015 Gew.-%ige wäßrige NaOH verwendet wird.

4. Verfahren gemäß einem der Ansprüche 1 bis 3, bei dem bei der kontinuierlichen Herstellung von Bis(4-hydroxyaryl)alkanen dem kristallin anfallenden und von der Mutterlauge befreiten Addukt ein konstanter Massenstrom von Alkalihydroxid zugeführt wird, das Addukt anschließend destillativ von der enthaltenenen aromatischen Hydroxyverbindung befreit, der Nebenproduktgehalt im erhaltenenen Bis(4-hydroxyaryl)alkan analytisch bestimmt und, falls der Gehalt bestimmter Nebenprodukte einen vorgegebenen Schwellenwert überschreitet, der Massenstrom von Alkalihydroxid entsprechend nachgeregelt wird.

5. Verfahren gemäß einem der Ansprüche 1 bis 4, bei dem das Aerosol auf der Druckseite einer Wasserringpumpe erzeugt wird, die mit verdünnter Alkalihydroxid-Lösung als Betriebsflüssigkeit betrieben wird.

6. Verfahren gemäß Anspruch 5, bei dem die Alkalihydroxid-Konzentration im Aerosol durch kontinuierliche mengengeregelte Zufuhr von Wasser und Alkalihydroxid-Lösung und kontinuierliche Ausschleusung eines Teils der Betriebsflüssigkeit geregelt wird.

7. Verwendung einer mit Alkalihydroxid-Lösung als Betriebsflüssigkeit betriebenen Wasserringpumpe zur Erzeugung von Alkalihydroxid enthaltenden Aerosolen in einem Verfahren gemäß einem der Ansprüche 1 bis 6.

## Claims

1. A process for producing bis(4-hydroxyaryl)alkanes from addition products of bis(4-hydroxyaryl)alkanes and aromatic hydroxy compounds which are obtained by the acid-catalysed reaction of the aromatic hydroxy compounds with ketones, wherein an aqueous alkaline solution is fed as an aerosol via the gas phase to the crystalline addition product and the addition product is subsequently freed from the aromatic hydroxy compound by distillation.

2. A process according to claim 1, wherein the addition product of a bis(4-hydroxy-aryl)alkane and an aromatic hydroxy compound is an addition product of bisphenol A and phenol.

3. A process according to claim 2, wherein 0.005 to 0.015 % by weight aqueous NaOH is used as the aqueous alkali hydroxide solution.

4. A process according to any one of claims 1 to 3, wherein during the continuous production of bis(4-hydroxyaryl)alkanes a constant mass flow of alkali hydroxide is fed to the addition product, which occurs in crystalline form and which is freed from mother liquor, the addition product is subsequently freed by distillation from the aromatic hydroxy compound which it contains, the content of by-products in the bis(4-hydroxyaryl)alkane obtained is determined by analysis, and if the content of defined by-products exceeds a predetermined threshold value the mass flow of alkali hydroxide is correspondingly readjusted.

5. A process according to any one of claims 1 to 4, wherein the aerosol is produced on the delivery side of a water ring pump which is operated using dilute alkali hydroxide solution as the operating liquid.

6. A process according to claim 5, wherein the alkali hydroxide concentration in the aerosol is controlled by a continuous, quantitatively controlled feed of water and alkali hydroxide solution and by the continuous removal of part of the operating liquid.

7. The use of a water ring pump which is operated with an alkali hydroxide solution as the operating liquid for the production of aerosols which contain alkali hydroxides in a process according to any one of claims 1 to 6.

## Revendications

1. Procédé de préparation de bis(4-hydroxy-aryl)alcanes à partir d'adduits de bis(4-hydroxy-aryl)alcanes et des composés hydroxylés aromatiques, lesquels sont obtenus par réaction sous catalyse acide des composés hydroxylés aromatiques avec des cétones, dans lequel une solution aqueuse d'hydroxyde alcalin est amenée en phase gazeuse sous forme d'un aérosol sur l'adduit cristallin, et l'adduit est ensuite libéré, par distillation, du composé hydroxylé aromatique.

2. Procédé suivant la revendication 1, dans lequel l'adduit du bis(4-hydroxyaryl)alcane et du composé hydroxylé aromatique est un adduit du bisphénol A et du phénol.

3. Procédé suivant la revendication 2, dans lequel on utilise comme solution aqueuse d'hydroxyde alcalin, du NaOH aqueux de 0,005 à 0,015% en poids.

4. Procédé suivant l'une quelconque des revendications 1 à 3, dans lequel lors de la préparation continue de bis(4-hydroxyaryl)alcanes, on amène sur l'adduit cristallin formé et libéré des liqueurs mères, un flux massique constant d'hydroxyde alcalin, l'adduit est ensuite libéré par distillation, du composé hydroxylé aromatique présent, la teneur en produits secondaires est déterminée de manière analytique dans le bis(4-hydroxyaryl)alcane obtenu et si la teneur en les produits secondaires dépasse une valeur seuil préindiquée, le flux massique d'hydroxyde alcalin est réglé de manière correspondante.

5. Procédé suivant l'une quelconque des revendications 1 à 4, dans lequel l'aérosol est produit du côté refoulement d'une pompe à anneau d'eau, qui est mise en fonctionnement avec une solution d'hydroxyde alcalin diluée comme liquide de fonctionnement.

6. Procédé suivant la revendication 5, dans lequel la concentration en hydroxyde alcalin est réglée dans l'aérosol, par amenée quantitative continue d'eau et de la solution d'hydroxyde alcalin et éclusage continu d'une partie du liquide de fonctionnement.

7. Utilisation d'un pompe à anneau d'eau fonctionnant avec une solution d'hydroxyde alcalin comme liquide de fonctionnement, pour produire un aérosol contenant de l'hydroxyde alcalin, dans un procédé selon l'une des revendications 1 à 6.
